# EUROPÄISCHE PATENTSCHRIFT

(11) **EP 2 854 891 B1**
(45) Veröffentlichungstag und Bekanntmachung des Hinweises auf die Patenterteilung: **28.08.2019**
(21) Anmeldenummer: 13726411.5
(22) Anmeldetag: 23.05.2013
(51) Int. Cl.: A61M 1/00

(54) **DRAINAGEBEHÄLTERVORRICHTUNG UND SAUGBEUTELEINHEIT**
DRAINAGE CONTAINER DEVICE AND A SUCTION POUCH UNIT
DISPOSITIF À RÉCIPIENT DE DRAINAGE ET UNITÉ DE SAC DE PRÉLÈVEMENT PAR ASPIRATION

(30) Priorität: 29.05.2012 CH 7382012
(43) Veröffentlichungstag der Anmeldung: 08.04.2015
(73) Patentinhaber: Medela Holding AG, 6340 Baar (CH)
(72) Erfinder: KOCH, Urs, CH-6404 Greppen (CH); EHLERT, Hilmar, CH-6052 Hergiswil (CH)
(74) Vertreter: Clerc, Natalia
(86) Internationale Anmeldenummer: PCT/CH2013/000089
(87) Internationale Veröffentlichungsnummer: WO 2013/177716

(56) Entgegenhaltungen:
- EP-A1- 0 861 668
- EP-A2- 0 882 440
- EP-B1- 1 225 930
- WO-A1-2008/144951
- US-A- 3 768 478
- US-A- 4 111 204
- US-A- 4 275 732

## Beschreibung

### TECHNISCHES GEBIET

Die vorliegende Erfindung betrifft eine Drainagebehältervorrichtung gemäss Oberbegriff des Patentanspruchs 1 und eine Saugbeuteleinheit gemäss Oberbegriff des Patentanspruchs 2.

### STAND DER TECHNIK

Im medizinischen Bereich werden Körperfluide mittels einer Vakuumquelle abgesaugt und in einer Drainagebehältervorrichtung gesammelt. Beispiele hierfür sind chirurgische Eingriffe, die Wunddrainage, die Thoraxdrainage oder das Absaugen von Körperfetten.

Es sind Drainagebehältervorrichtungen bekannt, welche einen starren Drainagebehälter und einen diesen dicht verschliessenden Deckel mit einem am Deckel befestigten flexiblen Saugbeutel aufweisen. Im Deckel ist ein patientenseitiger Drainageanschluss zur Verbindung mit einem zum Patienten führenden Drainageschlauch vorhanden. Ferner ist ein Sauganschluss vorhanden, welcher die Drainagebehältervorrichtung mit einer Saugquelle verbindet und welche durch Evakuierung des Saugbeutels einen Unterdruck in einer abzusaugenden Kavität des Patienten erzeugt. Der Sauganschluss kann im Deckel angeordnet und direkt mit dem Saugbeutel verbunden sein. Er kann auch im starren Drainagebehälter angeordnet sein, so dass zuerst der Drainagebehälter und erst dann der in ihm angeordnete Saugbeutel evakuiert werden. Hierfür kann im Deckel ein Verbindungskanal zwischen Behälterinnenraum und Beutelinnenraum vorhanden sein.

Ist ein Saugbeutel voll, wird zuerst der Drainageanschluss und, falls vorhanden, auch der Sauganschluss im Deckel gelöst. Anschliessend wird der Deckel zusammen mit dem Saugbeutel vom Behälter entfernt und entsorgt. Der Behälter kann nach Reinigung gemäss Krankenhausrichtlinien für denselben Patienten weiter verwendet werden. Ist der Sauganschluss im Behälter angeordnet, muss dieser nicht entfernt werden. Sauganschlüsse im Deckel weisen deshalb den Nachteil auf, dass beim Wechseln des Beutels bzw. beim Entleeren des Behälters sowohl die Drainageschlauchverbindung wie auch die Verbindung zur Vakuumquelle gelöst werden müssen. Dies ist einerseits zeitraubend und andererseits besteht die Gefahr, dass die Schläuche anschliessend mit den falschen Anschlüssen verbunden werden.

EP 1 225 930 offenbart eine Drainagebehältervorrichtung mit einem einstückigen Deckel und einem im Deckel verlaufenden Kanal, welcher zum Sauganschluss im starren Behälter führt. Ein Filter zum Schutz der Vakuumquelle ist an der dem Innenraum des Beutels zugewandten Unterseite des Deckels angeordnet, wobei das Filter dem Ende des Kanals angepasst ist. Das hier verwendete Filter weist einen u-förmigen Querschnitt auf und ist entsprechend kompliziert und teuer in der Herstellung.

WO 94/14045 beschreibt ebenfalls eine derartige Drainagebehältervorrichtung, wobei ein flaches Filter im mehrteiligen Deckel angeordnet ist. Dieser mehrteilige Deckel ist aufwendig in der Fertigung und Montage.

US 3 768 478 zeigt eine Drainagebehältervorrichtung mit einem Sauganschluss im Deckel und einem zum Behälterinnenraum führenden Kanal. Unterhalb des Sauganschlusses ist ein Hohlraum vorhanden zur Aufnahme eines porösen Pads, welches bei Kontakt mit Wasser anschwillt und zusammen mit einem darüber liegenden wasser- und luftdichten Pad als Überströmventil dient.

US 4 111 204 zeigt eine Drainagebehältervorrichtung mit einem Sauganschluss im Deckel. Der Deckel weist unterhalb des Sauganschlusses eine Kammer auf, in welche ein luftdurchlässiges und wasserundurchlässiges Filter eingelegt ist. Der Sauganschluss bildet eine obere Abdeckung dieser Kammer.

US 4 487 606 zeigt ebenfalls eine Drainagebehältervorrichtung mit einem Sauganschluss im Deckel, wobei hier ein Filter auf der Unterseite des Deckels befestigt und mit einer Schutzkappe überdeckt ist.

In US 4 460 361 ist auf der Unterseite des Deckels ein Überströmventil in einem vorstehenden Gehäuse angeordnet.

Filter, welche im Innern des Saugbeutels angeordnet sind, weisen den Nachteil auf, dass sie Spritzern der abgesaugten Körperflüssigkeit ausgesetzt sind und dadurch relativ schnell verstopfen. Dadurch reduziert sich die Saugleistung. Filter, welche in einer Kammer angeordnet sind, sind entsprechend geschützt.

US 6 261 276 offenbart einen beutellosen Behälter mit einer im Deckel integrierten manuellen Vakuumpumpe. Ein hydrophobes Filter ist auf der Aussenseite des Deckels angeordnet. Eine manuelle Vakuumpumpe eignet sich für Notfälle, jedoch nicht für einen Betrieb über längere Zeit.

### DARSTELLUNG DER ERFINDUNG

Es ist deshalb eine Aufgabe der Erfindung, eine Drainagebehältervorrichtung und eine Saugbeuteleinheit der eingangs genannten Art zu schaffen, welche ein kostengünstiges und einfach montierbares Schutzelement zum Vermeiden von Überlauf und/oder einer Verschmutzung der Vakuumquelle ermöglichen.

Diese Aufgabe löst eine Drainagebehältervorrichtung mit den Merkmalen des Patentanspruchs 1 sowie eine Saugbeuteleinheit mit den Merkmalen des Patentanspruchs 2.

Die erfindungsgemässe Drainagebehältervorrichtung zum Sammeln von abgesaugten Körperfluiden, insbesondere von Körperflüssigkeiten, mittels einer externen Vakuumquelle weist auf
einen Drainagebehälter mit einem Sauganschluss zur Verbindung mit der Vakuumquelle;
einen im Wesentlichen einstückigen Behälterdeckel zum Verschliessen und Öffnen des Behälters und mit einem Drainageanschluss zum Verbinden mit einer patientenseitigen Drainageleitung;
einen Saugbeutel zur Aufnahme des abgesaugten Körperfluids, welcher am Behälterdeckel angeordnet ist und vom Drainagebehälter aufgenommen ist;
einen Kanal, welcher mindestens teilweise durch den Behälterdeckel verläuft, welcher einen Innenraum des Saugbeutels über einen Innenraum des Drainagebehälters mit dem Sauganschluss verbindet und ansonsten geschlossen ist, und
ein Schutzelement zum Vermeiden von Überlauf und/oder einer Verschmutzung der Vakuumquelle durch abgesaugtes Körperfluid.
Erfindungsgemäss ist das Schutzelement im Kanal angeordnet und im Kanal ist eine Kammer vorhanden, in welcher das Schutzelement angeordnet ist.

Die erfindungsgemässe Saugbeuteleinheit zur Verwendung in einer derartigen Drainagebehältervorrichtung weist auf
einen im Wesentlichen einstückigen Behälterdeckel zum Verschliessen und Öffnen des Drainagebehälters und mit einem Drainageanschluss zum Verbinden mit einer patientenseitigen Drainageleitung;
einen Saugbeutel zur Aufnahme des abgesaugten Körperfluids, welcher am Behälterdeckel angeordnet ist zur Aufnahme im Drainagebehälter;
einen Kanal, welcher durch den Behälterdeckel verläuft und einen Innenraum des Saugbeutels mit einem Innenraum des Drainagebehälters verbindet zwecks Verbindung mit dem Sauganschluss, wobei der Kanal ansonsten geschlossen ist, und
ein Schutzelement zum Vermeiden von Überlauf und/oder einer Verschmutzung der Vakuumquelle durch abgesaugtes Körperfluid.
Erfindungsgemäss ist das Schutzelement im Kanal angeordnet und im Kanal ist eine Kammer vorhanden, in welcher das Schutzelement angeordnet ist.

Die Kammer ist von einer dem Saugbeutel abgewandten Aussenseite des Behälterdeckels her mit einer separaten Kammerabdeckung dicht verschlossen. Diese Kammerabdeckung kann mit dem Behälterdeckel verklebt, verschweisst, verlötet oder anderweitig dicht verbunden sein.

Das Schutzelement ist vorzugsweise ein luftdurchlässiges/wasserundurchlässiges und/oder bakterienhemmendes Filter. Es können auch mehrere Filter mit gleichen oder unterschiedlichen Eigenschaften in Absaugrichtung hintereinander angeordnet sein. Das Schutzelement kann alternativ oder zusätzlich ein Überströmventil sein. Beispielsweise kann ein Filtermaterial bei Kontakt mit einer Flüssigkeit anschwellen und so den Durchlass luftdicht verschliessen. Das Filtermaterial kann gleichzeitig bakterienhemmend ausgebildet sein. Im Folgenden wird der Begriff "Filter" für alle derartigen Typen von Schutzelementen verwendet. Das Schutzelement dient somit zum Vermeiden einer Verschmutzung der Vakuumquelle durch abgesaugtes Körperfluid und/oder zum Vermeiden von Überlauf des gefüllten Saugbeutels.

Die erfindungsgemässe Vorrichtung und die erfindungsgemässe Einheit kombinieren die Vorteile der behälterseitigen Absaugung ohne Sauganschluss im Deckel mit den Vorteilen der Anordnung des Filters in einer Kammer. Insbesondere lässt sich ein planes und flaches Filter verwenden. Zudem lassen sich mehrere Filter auf einfache Art und Weise in Absaugrichtung übereinander anordnen.

Vorzugsweise ist ein Spritzschutz vorhanden, welcher das Schutzelement gegenüber dem Innenraum des Saugbeutels vor Körperfluiden schützt. Ein frühzeitiges Verstopfen der Filteröffnungen ist verhindert, und die optimale Saugleistung ist bis zum Erreichen des maximalen Füllstandes gewährleistet. Ist die Kammer zum Beutelinnern hin bis auf Absaugöffnungen geschlossen, so schützt sie das Filtermaterial vor Spritzern. Der Spritzschutz kann zusätzlich oder alternativ auch durch Spritzschutzelemente gebildet sein, welche an einem zum Innenraum des Saugbeutels hin gerichteten Boden angeformt sind. In einer bevorzugten Ausführungsform ragen diese Spritzschutzelemente zum Innenraum des Saugbeutels hin, wobei sie eine nicht geradlinige Verbindung zwischen Innenraum und Kammer bilden. Vorzugsweise ist jede Absaugöffnung mit einem derartigen Spritzschutzelement versehen. Alternativ oder zusätzlich kann der Spritzschutz auch eine auf die Kammer aufsetzbare Schutzkappe sein, welche die Kammer überdeckt, aber beabstandet zu ihr angeordnet ist, so dass ein Strömungskanal zwischen Kammer und Schutzkappe vorhanden ist.

Der Spritzschutz lässt sich alternativ oder zusätzlich erhalten, indem der Drainageanschluss eine Tülle aufweist, welche in den Innenraum des Beutels hineinragt, wobei die Tülle eine Wandung aufweist, welche im Bereich der Kammer länger ausgebildet ist als im gegenüberliegenden Bereich. Dadurch werden Spritzer auf die der Kammer gegenüberliegende Seite des Beutels abgelenkt.

Die Kammer ist vorzugsweise Bestandteil des im Wesentlichen einstückig ausgebildeten Behälterdeckels. Sie kann jedoch auch am Behälterdeckel befestigt oder in eine entsprechende Ausnehmung des Deckels eingesteckt sein.

Ist die Kammer von der Oberseite des Behälterdeckels, also von der dem Beutelinnern abgewandten Seite des Behälterdeckels zugänglich, so ist die Montage erleichtert. Die Herstellungskosten des Behälterdeckels und der Kammer sind optimiert. Des Weiteren lässt sich dank dieser Anordnung die Saugbeuteleinheit auch erst nach der Befestigung des Saugbeutels am Behälterdeckel mit Filtern ausrüsten. Ein Nachrüsten der Einheit mit weiteren Filtern ist ebenfalls möglich.

Die Anordnung des Filters in einer Kammer weist auch den Vorteil auf, dass die Filtergrösse und die Filterform beliebig gewählt werden können. Insbesondere können relativ grosse Filterflächen verwendet werden, was die Saugleistung erhöht.

Vorzugsweise ist das Schutzelement plattenförmig ausgebildet. Vorzugsweise ist das Schutzelement, insbesondere, in seiner plattenförmigen Ausgestaltung, in die Kammer eingelegt. In einer bevorzugten Ausführungsform ist das Schutzelement, wiederum vorzugsweise in seiner plattenförmigen Ausgestaltung, durch Auflagedruck der Kammerabdeckung in seiner Position gehalten.

Vorzugsweise fluchtet eine Quermittelachse des Schutzelements mit einer Quermittelachse der Kammer. Das Schutzelement ist somit gerade und nicht schräg in der Kammer angeordnet.

Vorzugsweise ist die Kammer beabstandet zum Mittelpunkt des Behälterdeckels angeordnet. Dadurch lässt sich die Länge des Kanals minimieren.

In einer bevorzugten Ausführungsform weist die Kammer in ihrem Innern einen umlaufenden Vorsprung auf zur Auflage des Schutzelements, wobei der Vorsprung zu einer dem Innenraum abgewandten Aussenseite des Behälterdeckels zugewandt ist.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfindungsgemässen Drainagebehältervorrichtung mit Drainagebehälter und Behälterdeckel;
- Figur 2: eine perspektivische Darstellung einer erfindungsgemässen Saugbeuteleinheit zur beispielhaften Verwendung in der Drainagebehältervorrichtung gemäss Figur 1;
- Figur 3: einen Längsschnitt durch einen Behälterdeckel der Saugbeuteleinheit gemäss Figur 2 ohne Schutzkappe;
- Figur 4: einen Längsschnitt durch den Behälterdeckel der Saugbeuteleinheit gemäss Figur 2 mit Schutzkappe;
- Figur 5: eine Ansicht des Behälterdeckels gemäss Figur 3 von unten;
- Figur 6: eine perspektivische Darstellung des Behälterdeckels gemäss Figur 5 von unten;
- Figur 7: den Behälterdeckel gemäss Figur 6 mit Schutzkappe und
- Figur 8: einen Längsschnitt durch eine erfindungsgemässe Drainagebehältervorrichtung gemäss einer zweiten Ausführungsform der Erfindung.

### BESCHREIBUNG BEVORZUGTER AUSFÜHRUNGSFORMEN

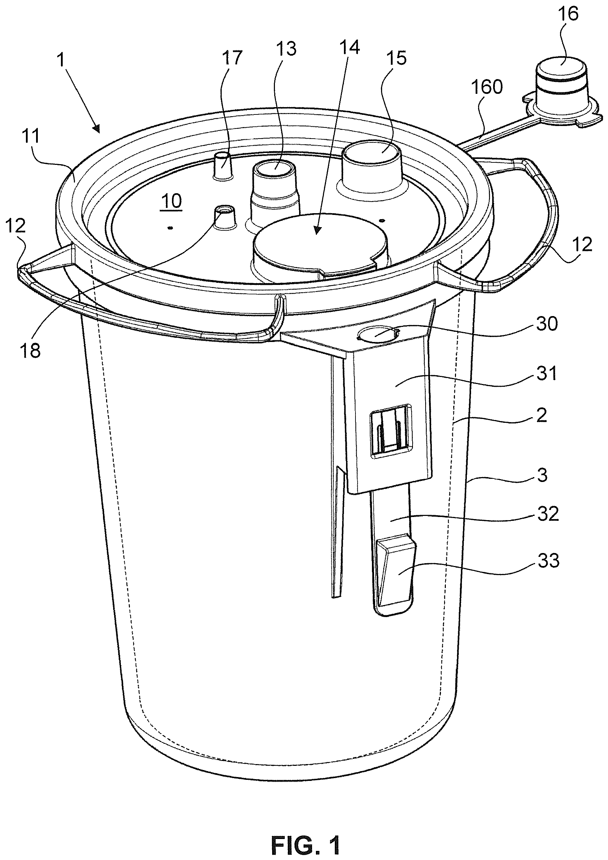

Figur 1 zeigt eine bevorzugte Ausführungsform einer erfindungsgemässen Drainagebehältervorrichtung. Sie umfasst einen starren Drainagebehälter 3, welcher einen Aussenbehälter bildet. Er ist vorzugsweise aus einem Kunststoff und insbesondere durchsichtig ausgebildet. Auf dem Drainagebehälter 3 ist ein Behälterdeckel 1 aufgesetzt bzw. in diesen eingesetzt. Der Behälterdeckel 1 verschliesst den Drainagebehälter 3 luftdicht. Vorzugsweise ist hierfür am Drainagebehälter 3 oder am Deckel 1 ein Dichtring vorhanden.

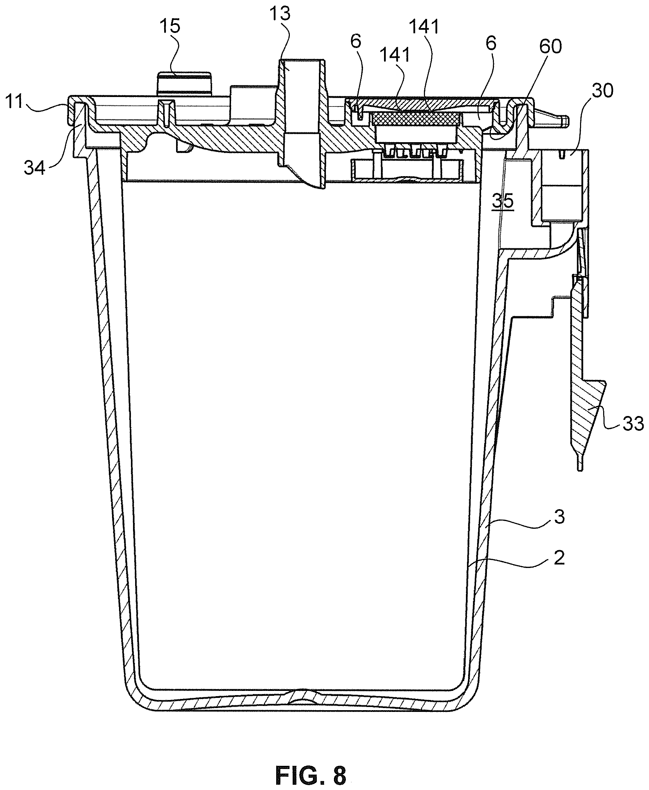

Der Deckel 1 weist in diesem Beispiel in seinem Rand 11 eine vollständig umlaufende, nach unten offene federnde Nut 110 auf, welche über einen Rand 34 (siehe Figur 8) des Behälters 3 stülpbar ist.

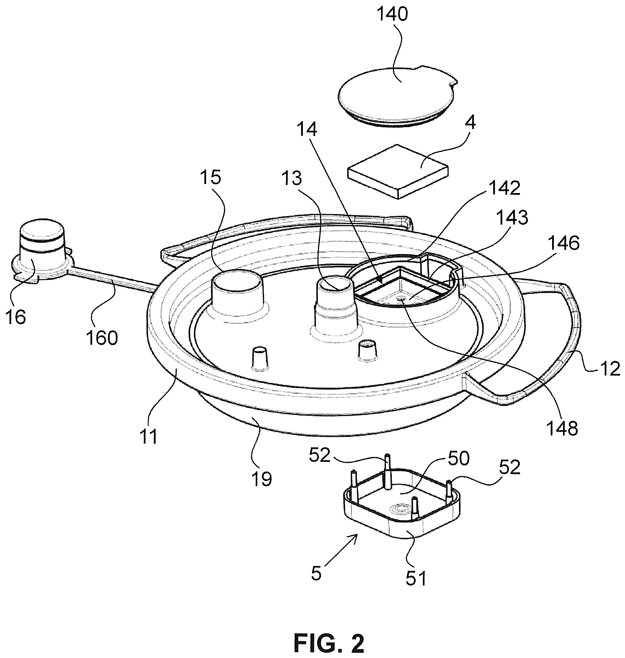

Am Deckel 1 ist ein flexibler Saugbeutel 2 befestigt, so dass er bei aufgesetztem Deckel 1 im Behälter 3 aufgenommen ist und gemeinsam mit dem Deckel 1 vom Behälter 3 entfernt werden kann. Der Saugbeutel 2 ist vorzugsweise am Deckel 1 angeklebt oder mit ihm verschweisst. Auf jeden Fall ist er vorzugsweise fest mit ihm verbunden. Der Deckel 1 weist hierfür vorzugsweise eine umlaufende Schürze 19 auf, wie sie in Figur 2 gut erkennbar ist. An einer äusseren oder inneren Fläche dieser Schürze 19 ist der Saugbeutel 2 vorzugsweise befestigt.

Am Behälter 3 ist ein Vakuumanschluss angeordnet, hier Sauganschluss 30 genannt, zur Verbindung mit einer nicht dargestellten externen Vakuumquelle. Vorzugsweise ist der Sauganschluss 30 einstückig gemeinsam mit dem restlichen Behälter 1 ausgebildet. Im hier dargestellten Ausführungsbeispiel befindet er sich in einem Befestigungsvorsatz 31, welcher der Wandung des Behälters 3 vorsteht und welcher zur Befestigung des Behälters 3 an einer Schiene dient. Zur Befestigung und einfachen Lösbarkeit weist dieser Befestigungsvorsatz 31 vorzugsweise ein Federelement 32 mit einer Rückhaltenase 33 auf.

Die externe Vakuumquelle kann beispielsweise eine mobile Vakuumpumpe oder ein zentrales Vakuumsystem eines Spitals sein. Der Sauganschluss ist vorzugsweise eine Öffnung oder ein vorstehender Stutzen, in welche bzw. auf welchen ein zur Vakuumquelle führender Schlauch ein- bzw. aufsteckbar ist.

Der Deckel 1 ist vorzugsweise im Wesentlichen einstückig ausgebildet. Vorzugsweise ist wenigstens der Grundkörper 10 einstückig ausgebildet. Einzelne Kleinteile und auch allfällige Dichtungselemente können beispielsweise separat ausgebildet sein. Der Deckel 1 besteht vorzugsweise aus Kunststoff. Er weist eine zur Öffnung des Behälters 3 passende Form auf. Hier ist er rund ausgebildet.

Im Deckel 1, genauer im Grundkörper 10, ist ein Drainageanschluss 13 zur Verbindung mit einem zum Patienten führenden Drainageschlauch vorhanden. Auch dieser Drainageanschluss 13 kann eine Öffnung sein. Vorzugsweise ist er jedoch ein Stutzen, welcher dem Deckel 1 nach aussen, d.h. vom Saugbeutel 2 abgewandt, vorsteht. Der Drainageanschluss 13 ist in dieser Ausführungsform mittig im runden Deckel 1 angeordnet. Er kann sich jedoch auch an einer anderen Stelle befinden.

Der Deckel 1 kann, wie in dieser Ausführungsform dargestellt, zusätzlich einen seriellen Anschluss 15 zur Verbindung dieser Drainagebehältervorrichtung mit einer zweiten ähnlich oder gleich ausgebildeten Drainagebehältervorrichtung aufweisen. Dieser Anschluss wird benötigt, wenn eine Absaugung über zwei oder mehrere Behälter in Serie erfolgt. Wird nur eine Vorrichtung verwendet, so ist dieser serielle Anschluss 15 mit einem Verschlussdeckel 16 verschlossen. Dieser Verschlussdeckel 16 ist vorzugsweise einstückig am Deckel 1 angeformt. Die entsprechende Verbindungslasche ist in Figur 1 mit dem Bezugszeichen 160 versehen.

Der Deckel 1 weist vorzugsweise Handgriffe 12 auf, um den Deckel 1 mit dem Saugbeutel 2 auf einfache Weise vom Behälter 3 zu entfernen. Die Handgriffe 12 sind vorzugsweise ebenfalls gemeinsam einstückig mit dem Grundkörper des Deckels 1 ausgebildet.

Am oder auf dem Deckel 1 können auch, wie hier dargestellt ist, Befestigungsstutzen 17, 18 angeformt sein, um weitere Teile zu befestigen. Diese Befestigungsstutzen 17, 18 können beispielsweise zur Befestigung von Verschlussdeckeln für den Drainageanschluss 13 und von einem Adapteranschluss für den Drainageschlauch dienen.

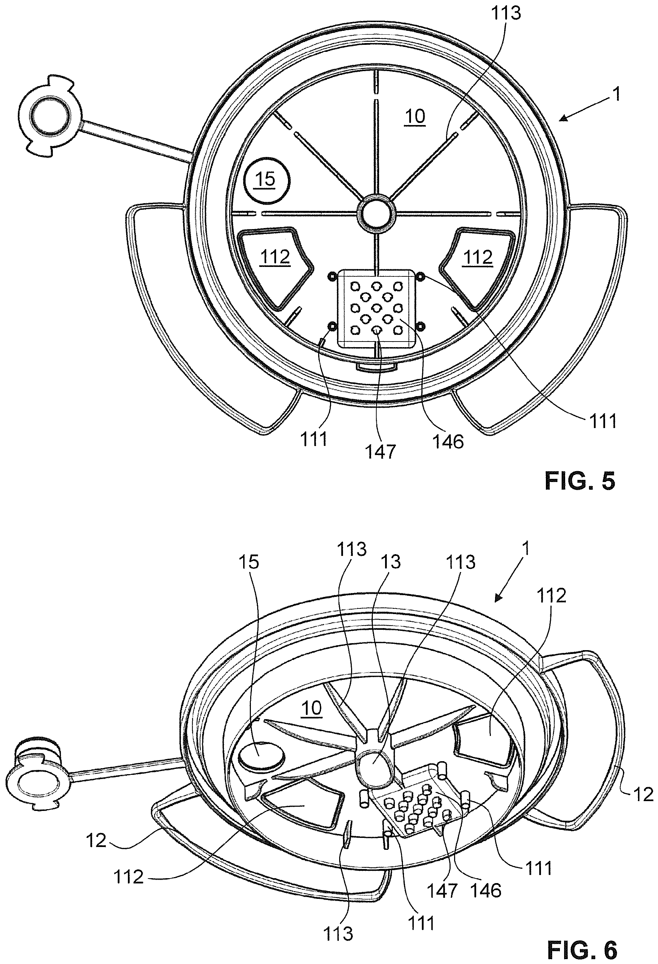

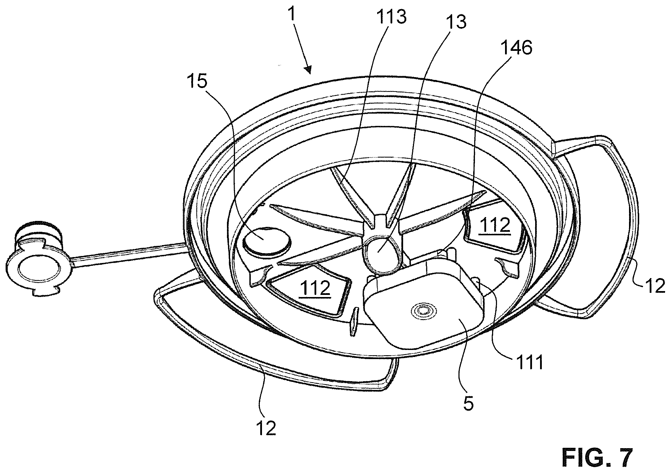

In den Figuren 5 bis 7 sind Entleerungsfenster 112 dargestellt, welche wahlweise im Deckel 1 vorhanden sind. Sie sind als ausbrechbare Teile im Grundkörper 10 ausgebildet und dienen zur Erstellung von Öffnungen, wenn der Saugbeutel 2 voll ist. Durch diese nachträglich ausbrechbaren Fenster 112 lässt sich der Saugbeutel 2 nach Entfernen aus dem Behälter 3 entleeren und der leere Saugbeutel 2 lässt sich anschliessend entsorgen. Es ist jedoch auch möglich, den Saugbeutel und Deckel mit abgesaugtem Inhalt zu entsorgen.

Ebenfalls in den Figuren 5 bis 7 sind Rippen 113 erkennbar, welche wahlweise vorhanden sein können und welche zur Verstärkung des Deckels 1 dienen.

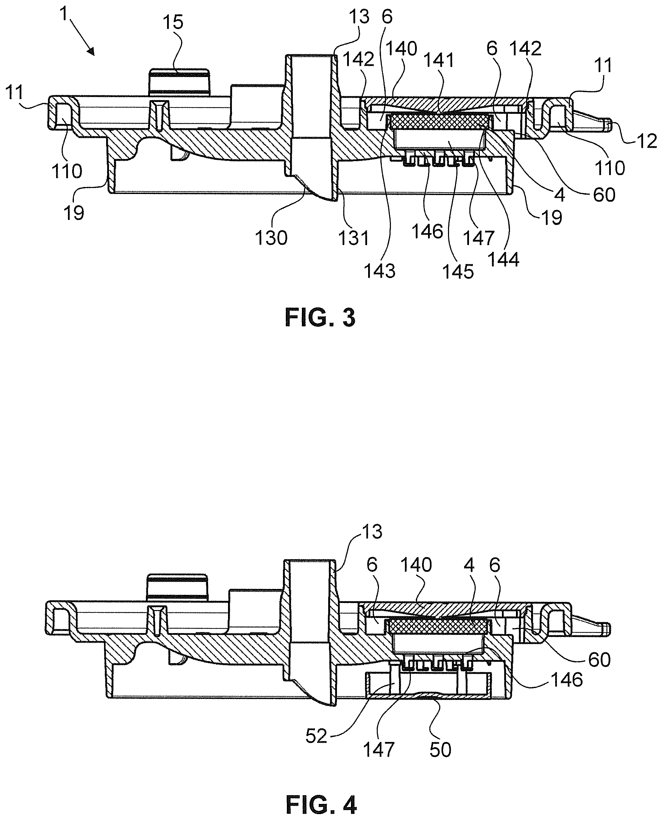

Im Deckel 1 ist ferner eine Kammer 14 angeordnet, welche zur Aufnahme eines Schutzelements, insbesondere eines Filters 4, dient. Dies ist in Figur 2 gut erkennbar. Die Kammer 14 ist vorzugsweise gemeinsam mit dem Grundkörper des Deckels 1 einstückig ausgebildet. Sie weist eine nach oben, d.h. vom Saugbeutel 2 abgewandte, offene Aufnahme auf. Diese Aufnahme ist von einer äusseren Wand 142 umgeben. In dieser Aufnahme befindet sich eine innere Wand 143, welche einen umlaufenden Vorsprung 144 aufweist. Dieser Vorsprung 144 bildet einen Sims zur Aufnahme des plattenförmigen Filters 4. Dies ist in Figur 3 gut erkennbar. Vorzugsweise ist die Kammer 14 beabstandet zum Mittelpunkt des Behälterdeckels 1 angeordnet.

Die Kammer 14 kann zum Saugbeutel 2 hin offen ausgebildet sein. Vorzugsweise weist sie jedoch einen zum Saugbeutel 2 hin gerichteten Boden 146 auf, in welchem Saugöffnungen 148 angeordnet sind. Ansonsten ist der Boden 146 vorzugsweise geschlossen ausgebildet. Die Saugöffnungen 148 verbinden den Innenraum des Beutels 2 mit der Kammer 14. Zwischen Filter 4 und Boden 146 kann, wie hier dargestellt, ein Hohlraum 145 vorhanden sein. Das Filter 4 kann aber auch auf dem Boden 146 aufliegen oder sich zumindest bis zum Boden hin erstrecken.

Das Schutzelement 4 ist mit seiner Fläche vorzugsweise senkrecht zur Strömungsrichtung angeordnet. Das heisst, es weist eine Quermittelachse auf, welche mit einer Quermittelachse der Kammer 14 fluchtet.

Es können auch anstelle eines einzigen Filters 4 zwei und mehrere Filter übereinander in der Kammer 14 angeordnet sein. Die Filter 4 können identisch sein und gleich wirken oder verschiedene Funktionen aufweisen. Das Filter 4 kann beispielsweise bakterienhemmend und/oder hydrophob und/oder bei Kontakt mit einer Flüssigkeit aufschwellend und luftdicht schliessend sein. Das Filter 4 kann aber auch ein Schutzfilter gegen Rauch sein. Die Filter sind luftdurchlässig ausgebildet, um ein Absaugen durch die Filter zu ermöglichen. Ist das Filter ein Überströmventil, d.h. zum Beispiel aufschwellend, blockiert es im geschlossenen Zustand den weiteren Luftdurchlass. Das Filter kann aus bekannten Materialien bestehen, beispielsweise aus einem Kunststoff, insbesondere aus einem porösen Polyethylen mit Quelleigenschaften.

Die Kammer 14 ist mit einer Kammerabdeckung 140 verschlossen. Diese lässt sich von der Aussenseite des Deckels 1 auf die Kammer 14 aufbringen. Vorzugsweise ist die Kammerabdeckung 140 mit der äusseren Wand 142 der Kammer 14 verschweisst, verlötet, verklebt oder anderweitig luftdicht verbunden. Wie in Figur 3 erkennbar ist, weist die Kammerabdeckung 140 vorzugsweise ein Fixierelement 141 auf, mit welchem das Filter 4 in seiner Position in der Kammer 14 fixiert ist. Die Fixierung erfolgt vorzugsweise flächig oder punktuell, vorzugsweise an wenigen Stellen, damit die luftdurchströmbare Fläche des Filters 4 maximiert ist.

In Figur 3 ist ein Kanal 6 erkennbar, welcher vom Innenraum des Saugbeutels 2 durch den Deckel 1 zum Drainagebehälter 3 verläuft. Er verläuft dabei durch die Saugöffnungen 148 im Boden 146 der Kammer 14, durch den Hohlraum 145, durch das Filter 4 in den Zwischenraum zwischen äusserer und innerer Wand 142, 143 bis zu einem behälterseitigen Ende 60. Dieses Ende 60 mündet in den Drainagebehälter 3 und stellt über den Hohlraum 35 (siehe Figur 8) eine Verbindung zum Sauganschluss 30 her. Der Kanal 6 ist bis auf die Saugöffnungen 148 und dem behälterseitigen Ende 60 geschlossen ausgebildet. Insbesondere hat er keine weitere Ausgangsöffnung aus dem Deckel 1 heraus, d.h. es gibt keinen Sauganschluss im Deckel.

Über diesen Kanal 6 lässt sich nun ein Unterdruck, welcher von der Vakuumquelle im Innenraum zwischen Saugbeutel 2 und Aussenwand des Behälters 3 angelegt wurde, auf den Innenraum des Saugbeutels 2 und von diesem über den Drainageanschluss 13 und die Drainageleitung auf eine abzusaugende Kavität eines Patienten übertragen. Körperfluide lassen sich so vom Patienten in den Saugbeutel 2 absaugen.

Um ein frühzeitiges Verstopfen des Filters 4 zu verhindern, ist vorzugsweise mindestens ein Spritzschutz vorhanden. Der Spritzschutz ist vorzugsweise durch eines oder mehrere der unten angeführten Merkmale gebildet:
- Der Drainageanschluss 13 weist vorzugsweise eine in den Innenraum des Saugbeutels 2 hinein ragende Tülle auf, deren beutelzugewandtes Ende 130 schräg ausgebildet ist. Hierbei ist die Tülle auf der dem Filter 4 zugewandten Seite verlängert ausgebildet. Das verlängerte Seitenteil ist in der Figur 3 mit dem Bezugszeichen 131 versehen.
- Die Saugöffnungen 148 im an sich geschlossenen Boden 146 der Kammer 14, insbesondere in Kombination mit dem zurückversetzen Filter 4, bildet ebenfalls bereits einen Spritzschutz.
- Vorzugsweise sind mehrere, vorzugsweise ist jede der Saugöffnungen, mit einem in den Innenraum des Saugbeutels 2 ragenden Spritzschutzelement 147 versehen. Dies sind kleine Kappen, welche am Boden 146 einstückig angeformt sind und welche einen nichtlinearen Zugang, vorzugsweise einen rechtwinkligen Zugang vom Saugbeutel 2 in die Kammer 14 schaffen. Diese Spritzschutzelemente 147 sind in den Figuren 3, 5 und 6 gut erkennbar.
- In der in den Figuren 2, 4 und 7 dargestellten Ausführungsform ist der Boden 146 der Kammer zusätzlich mit einer Schutzkappe 5 überdeckt, welche in den Innenraum des Saugbeutels 2 hineinragt. Die Schutzkappe 5 ist beabstandet zum Boden 146 angeordnet, so dass Luft durch den entstehenden Zwischenraum in die Kammer abgesogen werden kann. Die Schutzkappe 5 weist vorzugweise einen planen Boden 50 und einen senkrecht dazu ausgebildeten, den Boden 50 vollständig umlaufenden Mantel 51 auf. Zur Befestigung der Schutzkappe 5 sind Befestigungsstifte 52 vorhanden, welche in entsprechende Aufnahmen 111 des Behälterdeckels 1 einsteckbar sind. Die Aufnahmen 111 sind in Figur 5 erkennbar.

In Figur 8 sind die oben beschriebenen Teile nochmals in einer Zusammenschau dargestellt. Die Ausführungsform gemäss Figur 8 unterscheidet sich von der vorherigen lediglich dadurch, dass die Kammerabdeckung 140 nicht nur einen Punkt 141 sondern zwei oder mehrere aufweist. Diese sind in der Figur mit dem Bezugszeichen 141 dargestellt. Es kann auch ein Ring 141 vorhanden sein, welcher das Filter 4 fixiert.

Die erfindungsgemässe Vorrichtung und die erfindungsgemässe Einheit ermöglicht eine einfache Montage eines kostengünstigen Filters, wobei es in bevorzugten Ausführungsformen zudem einen Spritzschutz bietet.

**BEZUGSZEICHENLISTE**

| | | | |
|---|---|---|---|
| 1 | Deckel | 16 | Verschlussdeckel |
| | | 160 | Verbindungslasche |
| 10 | Grundkörper | | |
| | | 17 | erster Befestigungsstutzen |
| 11 | Rand | | |
| 110 | Nut | 18 | zweiter Befestigungsstutzen |
| 111 | Aufnahmen | | |
| 112 | Entleerungsfenster | 19 | Schürze |
| 113 | Rippe | | |
| | | | |
| 12 | Handgriff | 2 | Beutel |
| | | | |
| 13 | Drainageanschluss | 3 | Behälter |
| 130 | beutelzugewandtes Ende | 30 | Sauganschluss |
| 131 | verlängertes Seitenteil | 31 | Befestigungsvorsatz |
| | | 32 | Federelement |
| 14 | Kammer | 33 | Rückhaltenase |
| 140 | Kammerabdeckung | 34 | oberer Rand |
| 141 | Fixierelement | 35 | Innenraum |
| 142 | äussere Wand | | |
| 143 | innere Wand | 4 | Filter |
| 144 | Vorsprung | | |
| 145 | Hohlraum | 5 | Schutzkappe |
| 146 | Boden | 50 | Boden |
| 147 | Spritzschutzelemente | 51 | Mantel |
| 148 | Saugöffnungen | 52 | Befestigungsstifte |
| | | | |
| 15 | serieller Anschluss | 6 | Saugkanal |
| | | 60 | behälterseitiges Ende |

## Patentansprüche

1. Drainagebehältervorrichtung zum Sammeln von abgesaugten Körperfluiden mittels einer Vakuumquelle, wobei die Drainagebehältervorrichtung aufweist
einen Drainagebehälter (3) mit einem Sauganschluss (30) zur Verbindung mit der Vakuumquelle;
einen im Wesentlichen einstückigen Behälterdeckel (1), welcher auf den Drainagebehälter (3) aufsetzbar oder in diesen einsetzbar ist und zum Verschliessen und Öffnen des Drainagebehälters (3) dient, und welcher einen Drainageanschluss (13) zum Verbinden mit einer patientenseitigen Drainageleitung aufweist;
einen Saugbeutel (2) zur Aufnahme des abgesaugten Körperfluids, welcher am Behälterdeckel (1) angeordnet ist und vom Drainagebehälter (3) aufgenommen ist;
einen Kanal (6), welcher mindestens teilweise durch den Behälterdeckel (1) verläuft, welcher einen Innenraum des Saugbeutels (2) über einen Innenraum des Drainagebehälters (3) mit dem Sauganschluss (30) verbindet und ansonsten geschlossen ist, und
ein Schutzelement (4) zum Vermeiden von Überlauf und/oder einer Verschmutzung der Vakuumquelle durch abgesaugtes Körperfluid,
**dadurch gekennzeichnet,**
**dass** das Schutzelement (4) im Kanal (6) angeordnet ist, und dass im Kanal (6) eine Kammer (14) vorhanden ist, in welcher das Schutzelement (4) angeordnet ist, und welche von einer dem Saugbeutel (2) abgewandten Aussenseite des Behälterdeckels (1) her mit einer separaten Kammerabdeckung (140) dicht verschlossen ist.

2. Saugbeuteleinheit zur Verwendung in einer Drainagebehältervorrichtung gemäss Anspruch 1, wobei die Saugbeuteleinheit aufweist
einen im Wesentlichen einstückigen Behälterdeckel (1), welcher auf den Drainagebehälter (3) aufsetzbar oder in diesen einsetzbar ist und zum Verschliessen und Öffnen des Drainagebehälters (3) dient, und welcher einen Drainageanschluss (13) zum Verbinden mit einer patientenseitigen Drainageleitung aufweist;
einen Saugbeutel (2) zur Aufnahme des abgesaugten Körperfluids, welcher am Behälterdeckel (1) angeordnet ist zur Aufnahme im Drainagebehälter (3);
einen Kanal (6), welcher durch den Behälterdeckel (1) verläuft und einen Innenraum des Saugbeutels (2) mit einem Innenraum des Drainagebehälters (3) verbindet zwecks Verbindung mit dem Sauganschluss (30), wobei der Kanal (6) ansonsten geschlossen ist, und
ein Schutzelement (4) zum Vermeiden von Überlauf und/oder einer Verschmutzung der Vakuumquelle durch abgesaugtes Körperfluid,
**dadurch gekennzeichnet,**
**dass** das Schutzelement (4) im Kanal (6) angeordnet ist, und dass im Kanal (6) eine Kammer (14) vorhanden ist, in welcher das Schutzelement (4) angeordnet ist, und welche von einer dem Saugbeutel (2) abgewandten Aussenseite des Behälterdeckels (1) her mit einer separaten Kammerabdeckung (140) dicht verschlossen ist.

3. Saugbeuteleinheit nach Anspruch 2, wobei die Kammer (14) einen zum Innenraum des Saugbeutels (2) hin gerichteten Boden (146) aufweist mit Absaugöffnungen (148) zur Verbindung des Innenraums des Saugbeutels (2) mit dem Innenraum des Drainagebehälters (3).

4. Saugbeuteleinheit nach einem der Ansprüche 2 oder 3, wobei mindestens ein Spritzschutz vorhanden ist, welcher das Schutzelement (4) gegenüber dem Innenraum des Saugbeutels (2) vor Körperfluiden schützt.

5. Saugbeuteleinheit nach den Ansprüchen 3 und 4, wobei mindestens ein Teil der Absaugöffnungen (148) mit je einem zum Innenraum des Saugbeutels (2) ragenden Spritzschutzelement (147) versehen sind, wobei jedes Spritzschutzelement (147) eine nicht geradlinige Verbindung zwischen Innenraum und Kammer (14) bildet und als Spritzschutz wirkt.

6. Saugbeuteleinheit nach einem der Ansprüche 4 oder 5, wobei eine Schutzkappe (5) vorhanden ist, welche auf der dem Innenraum zugewandten Seite der Kammer (14) beabstandet zur Kammer (14) und die Kammer (14) überdeckend angeordnet ist und welche als Spritzschutz wirkt.

7. Saugbeuteleinheit nach einem der Ansprüche 2 bis 6, wobei die Kammer (14) im einstückigen Teil des Behälterdeckels (1) angeordnet ist.

8. Saugbeuteleinheit nach einem der Ansprüche 2 bis 7, wobei die Kammer (14) bis auf eine Kammerabdeckung (140) einstückig mit Behälterdeckel (1) ausgebildet ist.

9. Saugbeuteleinheit nach einem der Ansprüche 2 bis 8, wobei das Schutzelement (4) von dieser Aussenseite her in die Kammer (14) eingelegt ist.

10. Saugbeuteleinheit nach einem der Ansprüche 2 bis 9, wobei das Schutzelement (4) ein Filter und/oder ein Überströmventil ist.

11. Saugbeuteleinheit nach einem der Ansprüche 2 bis 10, wobei das Schutzelement (4) plattenförmig ausgebildet ist.

12. Saugbeuteleinheit nach einem der Ansprüche 2 bis 11, wobei eine Quermittelachse des Schutzelements (4) mit einer Quermittelachse der Kammer (14) fluchtet.

13. Saugbeuteleinheit nach einem der Ansprüche 2 bis 12, wobei die Kammer (14) beabstandet zum Mittelpunkt des Behälterdeckels (1) angeordnet ist.

14. Saugbeuteleinheit nach einem der Ansprüche 2 bis 10, wobei die Kammer (14) in ihrem Innern einen umlaufenden Vorsprung (144) aufweist zur Auflage des Schutzelements (4), wobei der Vorsprung (144) zu einer dem Innenraum des Saugbeutels (2) abgewandten Aussenseite des Behälterdeckels (1) zugewandt ist.

15. Saugbeuteleinheit nach einem der Ansprüche 2 bis 14, wobei der Drainageanschluss (13) eine Tülle aufweist, welche in den Innenraum des Saugbeutels (2) hineinragt, wobei die Tülle auf ihrer der Kammer (14) zugewandten Seite (131) länger ausgebildet ist als auf ihrer gegenüberliegenden Seite.

## Claims

1. A drainage container device for collecting suctioned bodily fluids by means of a vacuum source, wherein the drainage container device comprises:
a drainage container (3) with a suction port (30) to be connected with the vacuum source;
an essentially one-piece container lid (1) which can be placed on or in the drainage container (3) and serves for closing and opening the drainage container (3), and which comprises a drainage port (13) to be connected with a patient drainage line;
a suction bag (2) for holding the suctioned bodily fluid, which is arranged on the container lid (1) and accommodated by the drainage container (3);
a channel (6) which runs at least partially through the container lid (1), which joins an interior space of the suction bag (2) with the suction port (30) by way of an interior space of the drainage container (3), and is otherwise closed, and a protective element (4) for avoiding overflow and/or contamination of the vacuum source by a suctioned bodily fluid,
**characterized in that**
the protective element (4) is situated in the channel (14), and
the channel (6) incorporates a chamber (14) in which the protective element (4) is arranged and which is tightly sealed with a separate chamber cover (140) from an exterior side of the container lid (1) facing away from the suction bag (2).

2. A suction bag unit for use in a drainage container device according to claim 1, wherein the suction bag unit comprises:
an essentially one-piece container lid (1) which can be placed on or in the drainage container (3) and serves for closing and opening the drainage container (3), and which comprises a drainage port (13) to be connected with a patient drainage line;
a suction bag (2) for holding the suctioned bodily fluid, which is arranged on the container lid (1) for accommodation in the drainage container (3);
a channel (6) which runs through the container lid (1) and joins an interior space of the suction bag (2) with an interior space of the drainage container (3) for connection with the suction port (30), wherein the channel (6) is otherwise closed, and
a protective element (4) for avoiding overflow and/or contamination of the vacuum source by suctioned bodily fluid,
**characterized in that**
the protective element (4) is situated in the channel (6), and
the channel (6) incorporates a chamber (14) in which the protective element (4) is arranged and which is tightly sealed with a separate chamber cover (140) from an exterior side of the container lid (1) facing away from the suction bag (2).

3. The suction bag unit according to claim 2, wherein the chamber (14) comprises a floor (146) directed toward the interior space of the suction bag (2), with suction openings (148) for connecting the interior space of the suction bag (2) with the interior space of the drainage container (3).

4. The suction bag unit according to one of claims 2 or 3, wherein at least one splashguard is present, which safeguards the protective element (4) against bodily fluids in relation to the interior space of the suction bag (2).

5. The suction bag unit according to claims 3 and 4, wherein at least part of the suction openings (148) are each provided with a safeguard element (147) that projects toward the interior space of the suction bag (2), wherein each splashguard element (147) forms a nonlinear connection between the interior space and the chamber (14), and acts as a splashguard.

6. The suction bag unit according to one of claims 4 or 5, wherein a protective cap (5) is present, which, on the side of the chamber (14) facing the interior space is spaced apart from the chamber (14), covers the chamber (14), and acts as a splashguard.

7. The suction bag unit according to one of claims 2 to 6, wherein the chamber (14) is situated in the one-piece part of the container lid (1).

8. The suction bag unit according to one of claims 2 to 7, wherein the chamber (14) is designed as a single piece with a container lid (1), except for a chamber cover (140).

9. The suction bag unit according to one of claims 2 to 8, wherein the protective element (4) is inserted into the chamber (14) from this exterior side.

10. The suction bag unit according to one of claims 2 to 9, wherein the protective element (4) is a filter and/or an overflow valve.

11. The suction bag unit according to one of claims 2 to 10, wherein the protective element (4) is plate-shaped in design.

12. The suction bag unit according to one of claims 2 to 11, wherein a transverse central axis of the protective element (4) aligns with a transverse central axis of the chamber (14).

13. The suction bag unit according to one of claims 2 to 12, wherein the chamber (14) is spaced apart from the midpoint of the container lid (1).

14. The suction bag unit according to one of claims 2 to 10, wherein the interior of the chamber (14) comprises a continuous projection (144) to support the protective element (4), wherein the projection (144) faces an exterior side of the container lid (1) facing away from the interior space of the suction bag (2).

15. The suction bag unit according to one of claims 2 to 14, wherein the drainage port (13) comprises a spout that projects into the interior space of the suction bag (2), wherein the spout is longer on its side (131) facing the chamber (14) than on its opposite side.

## Revendications

1. Un dispositif à récipient de drainage pour recueillir des liquides corporels aspirés au moyen d'une source de vide, où le dispositif à récipient de drainage comporte un récipient de drainage (3) pourvu d'un raccord d'aspiration (30) pour établir une liaison avec la source de vide;
un couvercle de récipient (1) réalisé sensiblement en une seule pièce, pouvant être posé sur le récipient de drainage (3) ou inséré dans celui-ci et servant à ouvrir et à fermer le récipient de drainage (3), et lequel présente un raccord de drainage (13) pour établir une liaison avec une conduite de drainage située côté patient;
un sac d'aspiration (2) destiné à recueillir le fluide corporel aspiré, qui est disposé sur le couvercle de récipient (1) et logé dans le récipient de drainage (3);
un canal (6), qui traverse au moins en partie le couvercle de récipient (1), qui relie une poche intérieure du sac d'aspiration (2) au raccord d'aspiration (30) via un espace intérieur du récipient de drainage (3) et qui est sinon fermé; et
un élément de protection (4) pour éviter que le fluide corporel aspiré ne déborde et/ou ne salisse la source de vide, **caractérisé en ce que**
l'élément de protection (4) est situé dans le canal (6), et que une chambre (14) est située dans le canal (6), dans laquelle l'élément de protection (4) est disposé, et laquelle est fermée à l'aide d'un couvercle de chambre (140) à part de manière étanche depuis un côté extérieur du couvercle de récipient (1) et éloigné du sac d'aspiration (2).

2. Une unité de sac d'aspiration pour utilisation dans un dispositif à récipient de drainage selon la revendication 1, où l'unité de sac d'aspiration présente
un couvercle de récipient (1) réalisé sensiblement en une seule pièce, pouvant être posé sur le récipient de drainage (3) ou inséré dans celui-ci, et servant à ouvrir et à fermer le récipient de drainage (3), et lequel présente un raccord de drainage (13) pour établir une liaison avec une conduite de drainage située côté patient;
un sac d'aspiration (2) destiné à recueillir le fluide corporel aspiré, qui est disposé sur le couvercle de récipient (1) pour être logé dans le récipient de drainage (3);
un canal (6) qui traverse le couvercle de récipient (1) et qui relie une poche intérieure du sac d'aspiration (2) à un espace intérieur du récipient de drainage (3) afin d'établir une liaison au raccord d'aspiration (30), où le canal (6) est sinon fermé; et
un élément de protection (4) pour éviter que le fluide corporel aspiré ne déborde et/ou ne salisse la source de vide, **caractérisé en ce que**
l'élément de protection (4) est situé dans le canal (6) et que une chambre (14) est située dans le canal (6), dans laquelle l'élément de protection (4) est disposé, et laquelle est fermée à l'aide d'un couvercle de chambre (140) à part de manière étanche depuis un côté extérieur du couvercle de récipient (1) éloigné du sac d'aspiration (2).

3. Une unité de sac d'aspiration selon la revendication 2, où la chambre (14) présente un fond (146) orienté vers la poche intérieure du sac d'aspiration (2) avec des ouvertures d'aspiration (148) pour établir une liaison de la poche intérieure du sac d'aspiration (2) avec l'espace intérieur du récipient de drainage (3).

4. Une unité de sac d'aspiration selon une des revendications 2 ou 3, où au moins une protection d'éclaboussures est présente, laquelle protège l'élément de protection (4) par rapport à la poche intérieure du sac d'aspiration (2) de fluides corporels.

5. Une unité de sac d'aspiration selon les revendications 3 et 4, où au moins une partie des ouvertures d'aspiration (148) est munie respectivement d'un élément de protection d'éclaboussures (147) faisant saillie en direction de la poche intérieure du sac d'aspiration (2), où chaque élément de protection d'éclaboussures (147) forme une liaison non-rectiligne entre la poche intérieure et la chambre (14) et fonctionne en tant que protection d'éclaboussures.

6. Une unité de sac d'aspiration selon une des revendications 4 ou 5, où un capuchon de protection (5) est présent, lequel est disposé à distance de la chambre (14) sur le côté faisant face à l'espace intérieure de la chambre (14) et de manière recouvrant la chambre (14) et lequel fonctionne en tant que protection d'éclaboussures.

7. Une unité de sac d'aspiration selon une des revendications 2 à 6, où la chambre (14) est disposée dans la partie réalisée sensiblement en une seule pièce du couvercle de récipient (1).

8. Une unité de sac d'aspiration selon une des revendications 2 à 7, où la chambre (14) est réalisée sensiblement en une pièce avec le couvercle de récipient, mis à part le couvercle de chambre (140).

9. Une unité de sac d'aspiration selon une des revendications 2 à 8, où l'élément de protection (4) est inséré depuis le côté extérieur dans la chambre (14).

10. Une unité de sac d'aspiration selon une des revendications 2 à 9, où l'élément de protection (4) est un filtre et/ou une soupape de décharge.

11. Une unité de sac d'aspiration selon une des revendications 2 à 10, où l'élément de protection (4) est formé en plaque.

12. Une unité de sac d'aspiration selon une des revendications 2 à 11, où un axe central transversal de l'élément de protection (4) est aligné avec un axe central transversal de la chambre (14).

13. Une unité de sac d'aspiration selon une des revendications 2 à 12, où la chambre (14) est disposée de manière espacée par rapport au centre du couvercle de récipient (1).

14. Une unité de sac d'aspiration selon une des revendications 2 à 10, où la chambre (14) présente dans son intérieur une saillie en circonférence (144) pour y poser l'élément de protection (4), où la saillie (144) est orientée vers un côté extérieur du couvercle de récipient (1) éloigné de la poche intérieure du sac d'aspiration (2).

15. Une unité de sac d'aspiration selon une des revendications 2 à 14, où le raccord de drainage (13) présente un bec, lequel bec fait saillie vers la poche intérieure du sac d'aspiration (2), où le bec est réalisé de manière plus longue sur le côté (131) faisant face à la chambre (14) que sur le côté opposé.
